(19)

Europäisches
Patentamt

European
Patent Office

Office européen
des brevets

(11)  **EP 4 162 931 A1**

(12)  **EUROPEAN PATENT APPLICATION**

(43) Date of publication:
**12.04.2023  Bulletin 2023/15**

(21) Application number: **22207478.3**

(22) Date of filing: **13.03.2014**

(51) International Patent Classification (IPC):
**A61K 9/70** *(2006.01)*  **A61K 47/10** *(2006.01)*
**A61K 47/32** *(2006.01)*  **A61K 31/192** *(2006.01)*

(52) Cooperative Patent Classification (CPC):
**A61K 9/7053; A61K 31/192; A61K 47/10;
A61K 47/32**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.03.2013  US 201361778620 P**

(62) Document number(s) of the earlier application(s) in
accordance with Art. 76 EPC:
**14719442.7 / 2 968 201**

(71) Applicant: **Avery Dennison Corporation
Mentor, OH 44060 (US)**

(72) Inventors:
• **Carty, Neal
Mentor, 44060 (US)**
• **Wibaux, Anne Marie
Cleveland Heights (US)**

(74) Representative: **Müller-Boré & Partner
Patentanwälte PartG mbB
Friedenheimer Brücke 21
80639 München (DE)**

Remarks:
This application was filed on 15-11-2022 as a
divisional application to the application mentioned
under INID code 62.

(54)  **ENHANCED DRUG DELIVERY FROM ADHESIVES**

(57)  The present invention relates to an adhesive composition comprising: 20% to 90% by weight of an adhesive; 0.1% to 50% by weight of at least one absorbent; 0.1% to 20% by weight of at least one active agent; and 0% to 30% by weight of at least one crystallization inhibitor. In further aspects, the present invention relates to an article adapted to be adhered to biological skin and release at least one active agent to the skin, to a method of releasing at least one active agent to a region of interest on biological skin, and to a method of increasing at least one of (i) extent of release and (ii) rate of release of at least one active agent from the adhesive composition.

**EP 4 162 931 A1**

**Description**

**CROSS REFERENCES TO RELATED APPLICATIONS**

The present application claims the benefit of U.S. Provisional Application No. 61/778,620 filed March 13, 2013, which is incorporated herein by reference in its entirety.

**FIELD**

The present subject matter relates to adhesive compositions that release one or more active agents. The subject matter is particularly directed to medical adhesives containing one or more pharmaceutical active agents. More particularly, the present subject matter relates to improving release and release characteristics of actives from adhesive compositions.

**BACKGROUND**

Adhesive compositions are known which release one or more active agents. Although satisfactory in certain respects, some compositions exhibit unpredictable variations in the rate of release of the active(s). In addition, some compositions are not biologically compatible and so should not be left in contact with biological skin or tissue for an extended period of time, such as for example more than 24 hours. Accordingly, a need exists for improved adhesive compositions which exhibit predictable release characteristics, are biologically compatible, and which can be readily tailored and utilized in a variety of different applications.

**SUMMARY**

The difficulties and drawbacks associated with previously known compositions are addressed by certain compositions, articles, and related methods as follows. Generally, the present subject matter relates to improving drug or active agent delivery, rate of delivery, and/or delivery characteristics by incorporation of one or more absorbents in an adhesive composition.

In one aspect, the present subject matter provides a method of releasing or improving release of at least one active agent to a region of interest on biological skin. The method comprises providing an article adapted for placement along biological skin. The article defines at least one face. The method also comprises providing an adhesive composition including an adhesive component, 0.1% to 50% of at least one absorbent, and 0.1% to 20% of at least one active agent. The method also comprises depositing the adhesive composition onto the face of the article. The method additionally comprises applying the article to a region of interest on biological skin, whereby the adhesive composition is directed toward and overlies the region of interest. As described herein, incorporating an absorbent in the adhesive composition containing the active agent, results in improved release and/or release characteristics of the active agent from the composition.

In another aspect, the present subject matter provides a method of increasing at least one of the (i) extent of release, and (ii) rate of release, of at least one active agent from an adhesive composition including an adhesive component and at least one active agent. The method comprises incorporating at least one absorbent in the adhesive composition. The extent and/or the rate of release of the active agent is increased. In certain versions of the present subject matter, the extent of release and/or the rate of release of an active from a composition already including an absorbent can be increased by incorporating additional amounts of absorbent into the composition.

In another aspect, the present subject matter provides an adhesive composition comprising 20% to 90% of an adhesive, 0.1% to 50% of at least one absorbent, 0.1% to 20% of at least one active agent(s), 0.1% to 30% of at least one crystallization inhibitor, and 0.1% to 30% of a vehicle.

In another aspect, the present subject matter provides an adhesive composition comprising 30% to 80% of a hot melt adhesive, 0.5% to 45% of at least one absorbent, 0.5% to 10% of at least one active agent, 0.5% to 20% of at least one crystallization inhibitor, and 0.5% to 20% of at least one polyhydric alcohol.

In yet another aspect, the present subject matter provides an article adapted to be adhered to biological skin and release at least one active to the skin. The article defines a surface and comprises an adhesive composition disposed on at least a portion of the surface. The adhesive composition comprises 20% to 90% of an adhesive, 0.1% to 50% of at least one absorbent, 0.1% to 20% of at least one active agent(s), 0.1% to 30% of at least one crystallization inhibitor, and 0.1% to 30% of a vehicle.

In yet another aspect, the present subject matter also provides an article adapted to be adhered to biological skin and release at least one active to the skin. The article defines a surface and comprises an adhesive composition disposed on at least a portion of the surface. The adhesive composition comprises 30% to 80% of a hot melt adhesive,

0.5% to 45% of at least one absorbent, 0.5% to 10% of at least one active agent, 0.5% to 20% of at least one crystallization inhibitor, and 0.5% to 20% of at least one polyhydric alcohol.

In still another aspect, the present subject matter provides a method of forming an adhesive composition which releases at least one active agent. The method comprises providing an adhesive component including an adhesive and a vehicle. The method also comprises incorporating 0.1% to 50% of at least one absorbent, 0.1% to 20% of at least one active agent, and 0.1% to 30% of at least one crystallization inhibitor into the adhesive component to thereby form an adhesive composition which releases the at least one active agent.

As will be realized, the subject matter is capable of other and different embodiments and its several details are capable of modifications in various respects, all without departing from the subject matter. Accordingly, the drawings and description are to be regarded as illustrative and not restrictive.

## BRIEF DESCRIPTION OF THE DRAWINGS

Figure 1 is a graph of solubility of an active agent as a function of a ratio of two components in compositions according to an embodiment of the present subject matter.

Figure 2 is a graph of flux of an active agent from various compositions described in greater detail herein, several of which are in accordance with embodiments of the present subject matter.

Figure 3 is a graph of recovery of an active agent from several compositions described in greater detail herein and in accordance with embodiments of the present subject matter.

Figures 4 and 5 are graphs of elution of an active agent from several compositions described in greater detail herein and in accordance embodiments with the present subject matter.

## DETAILED DESCRIPTION OF THE EMBODIMENTS

In many applications, it is desirable to incorporate a drug or other active agents into an adhesive matrix to combine the therapeutic effects of the drug or active ingredient(s) with the cushioning, moisture-absorbing, and protective properties of the adhesive. The present subject matter relates to incorporating one or more active agents into an adhesive and particularly into a hydrocolloid adhesive. As described herein, it has been discovered that incorporation of one or more absorbents and/or absorbent agents in an adhesive composition in particular proportions and/or in conjunction with certain other agents, improves release and/or characteristics thereof of one or more actives, active agents, drugs, and/or pharmaceuticals from the adhesive composition.

Several aspects of the present subject matter, periodically referred to herein as a "drug-in-adhesive" system, include, but are not limited to the following. The active agents are controllably released from the adhesive composition into a receiving medium such as for example adjacent or underlying tissue. The active agents exist in a stable, amorphous state, i.e. noncrystalline state, within the adhesive composition. Crystallization of the active agents can cause unpredictable variations in the rate of active agent release or drug delivery, and therefore, such instability is to be avoided. The adhesive compositions exhibit good adhesive properties so that the compositions can remain on the skin for an extended period of time, for example 24 hours or more.

Generally, the present subject matter provides for certain combinations of adhesive, absorbent, drug or active agent, crystallization inhibitor (optionally), and vehicle to provide the noted aspects. The resulting adhesive compositions provide a drug-release feature without compromising absorption or adhesion performance. A representative example of an active agent is ibuprofen, although the subject matter is not limited to use solely with this active ingredient. Instead, as described in greater detail herein, a wide array of active agents or combinations of active agents can be incorporated in the present subject matter compositions.

The present subject matter provides an extensive range of adhesive compositions which can be tailored to controllably release one or more active agent(s) from the adhesive composition. Table 1 set forth below lists representative adhesive compositions and their components in accordance with the present subject matter. All percentages expressed herein are percentages by weight, unless noted otherwise.

Table 1 - Adhesive Compositions

| Weight Percentage | Component |
| --- | --- |
| 20% to 90% | Adhesive |
| 0.1% to 50% | Absorbent |
| 0.1% to 20% | Active Agent(s) |

(continued)

| Weight Percentage | Component |
|---|---|
| 0% to 30% | Crystallization Inhibitor |
| 0.1% to 30% | Vehicle |

Adhesive(s)

A wide array of adhesive components can be used in the adhesive compositions according to the present subject matter. Generally, the adhesive or adhesive component is a hot melt adhesive. In certain aspects, the present subject matter compositions comprise a hot melt pressure sensitive adhesive.

The adhesive matrix may be based on for example polyisobutylene, butyl rubber, polyacrylates, polyurethanes, silicone gum, natural gum rubber, SBR rubber or polyvinyl ether. Thermoplastic elastomers such as styrene-isoprene-styrene block copolymers and styrene-ethylene/propylene-styrene block copolymers may be used, and these may require optional tackifiers and plasticisers. Blends or mixtures of elastomers may be more easily employed.

Particularly suitable as bases for the pressure sensitive adhesives of the present subject matter are rubbers such as linear or radial A-B-A block copolymers or mixtures of these A-B-A block copolymers with simple A-B block copolymers. These block copolymers can be based on styrenebutadiene, styrene-isoprene, and hydrogenated styrene-diene copolymers such as styrene ethylene-butylene.

Suitable styrene-diene copolymers for the practice of the present subject matter are exemplified by a blend of linear styrene/isoprene/styrene triblock copolymer and linear styrene/isoprene diblock copolymer. Such a material is available from Shell Chemical as Kraton D-1161 and has a bound styrene content of about 15% and a diblock content of 17%. A second example is a blend of linear styrene/isoprene/styrene triblock copolymer and linear styrene/isoprene diblock copolymer available from Shell Chemical as Kraton D-1117 and which has a bound styrene content of about 17% and a diblock content of 33%.

An example of a suitable hydrogenated styrene-diene copolymer is a thermoplastic elastomer comprising a blend of clear linear triblock and diblock copolymer based on styrene and ethylene/butylene, with a bound styrene of 14% mass. Such a material is commercially available from Shell Chemical Company as Kraton G-1657. Another example is Kraton G-1652 from Shell Chemical Company, which is a thermoplastic elastomer comprised of a clear linear triblock copolymer based on styrene and ethylene-butylene, S-E/B-S, with a bound styrene content of about 30% by weight. Also suitable are polymers in which there is a combination of chemically saturated blocks and chemically unsaturated blocks. For example, a branched copolymer consisting of two polyisoprene chains attached to the rubber midblock of a styrene/ethylene-butylene/styrene triblock copolymer may be suitable. Such a material is available from Shell Chemical Company as Kraton Research Product RP6919. This material has a styrene content of 18%, an isoprene content of 36% and an ethylene-butylene content of 46% by weight. Also, a low styrene synthetic copolymer of butadiene and styrene, commonly called SBR rubber, can be used as a solid rubber.

Also particularly suitable are acrylic pressure sensitive adhesives, exemplified by an acrylic hot melt adhesive manufactured by Schenectedy Chemicals and having the designation Durotac 401. Another example is an acrylic solvent adhesive from Avery Chemicals called Polytex 7600.

Absorbent(s)

Similarly, a wide array of absorbents can be utilized in the adhesive compositions according to the present subject matter. Generally, the absorbent includes one or more hydrophilic polymers that are soluble or insoluble but swellable in water, as the moisture-absorbing component. Suitable insoluble swellable polymers include cross-linked sodium carboxymethyl cellulose, crystalline sodium carboxymethyl cellulose, cross-linked dextran and starch-acrylonitrile graft copolymer. The swellable polymer may also be a so-called "super absorbent" material such as starch sodium polyacrylate. Other hydratable polymers such as gluten and polymers of methyl vinyl ether and maleic acid and derivatives thereof may also be included. Suitable water soluble polymers include sodium carboxymethyl cellulose, pectin, gelatine, guar gum, locust bean gum, collagen, tragacanth gum, karaya gum starches, gum arabic, alginic acid and various sodium and/or calcium salts thereof. Other synthetic absorbents such as polyvinyl alcohol, polyvinyl acetate, polyvinyl pyrollidone, polyacrylic acid, polyhydroxyalkyl acrylates, polyacrylamides, high molecular weight polyethylene glycols and polypropylene glycols may be useful.

The super absorbent polymer (SAP), if used in the adhesive compositions, comprises a water-swellable, hydrogel-forming absorbent polymer capable of absorbing large quantities of liquids such as water, body fluids (e.g., urine, blood), and the like. Additionally, the SAP is capable of retaining such absorbed fluids under moderate pressures.

Typically the SAP absorbs many times its own weight in water, preferably at least 50 times, more preferably at least 100 times, most preferably at least 150 times its weight in water. Additionally, the SAP exhibits good saline fluid absorption under load and high saline fluid absorption capacity. Typically the SAP absorbs at least 10 times, preferably at least 30 times, more preferably at least 50 times its weight in saline fluid. Even though the SAP is capable of absorbing many times its own weight in water and/or saline, it does not dissolve in these fluids.

The ability of the SAP to absorb water and/or saline fluid is related to the degree of crosslinking present in the SAP. Increasing the degree of crosslinking increases the SAP's total fluid holding capacity under load. The degree of crosslinking is preferably optimized to obtain a composition in which the rate and amount of absorbency are optimized. Preferred SAPs are at least 10%, more preferably from about 10% to about 50%, and most preferably from about 20% to 40% crosslinked. Examples of suitable SAPs include crosslinked and polymerized $\alpha,\beta$-beta ethylenically unsaturated mono- and dicarboxylic acids and acid anhydride monomers including, e.g., acrylic acid, methacrylic acid, crotonic acid, maleic acid/anhydride, itaconic acid, fumaric acid, and combinations thereof.

Super absorbent polymers useful in the present subject matter include, e.g., crosslinked acrylate polymers, crosslinked products of vinyl alcohol-acrylate copolymers, crosslinked products of polyvinyl alcohols grafted with maleic anhydride, cross-linked products of acrylate-methacrylate copolymers, crosslinked saponification products of methyl acrylate-vinyl acetate copolymers, crosslinked products of starch acrylate graft copolymers, crosslinked saponification products of starch acrylonitrile graft copolymers, crosslinked products of carboxymethyl cellulose polymers and crosslinked products of isobutylene-maleic anhydride copolymers, and combinations thereof.

The super absorbent polymer(s) is typically in the form of particles and preferably are spherical and have an average particle size of from about 1 micrometer ($\mu$m) to about 400 ($\mu$m). Preferably the particles have an average particle size of from about 20 $\mu$m to about 200 $\mu$m, and more preferably from 20 $\mu$m to 150 $\mu$m. In one embodiment, the particle size of the particles is less than 150 $\mu$m, or less than 100 $\mu$m. Useful commercially available super absorbent particles include, e.g., sodium polyacrylate super absorbent particles available under the AQUA KEEP series of trade designations including, e.g., particles having an average particle size of from about 20 $\mu$m to about 30 $\mu$m available under the trade designation AQUA KEEP 1 OSH-NF, particles having an average particle size of from 200 $\mu$m to 300 $\mu$m available under the trade designation AQUA KEEP 10SH-P, particles having an average particle size of from 320 $\mu$m to 370 $\mu$m available under the trade designation AQUA KEEP SA60S, particles having an average particle size of from 350 $\mu$m to 390 $\mu$m available under the trade designations AQUA KEEP SA60SX, SA55SX $\pi$ and SA 60SL II, and particles having an average particle size of from 250 $\mu$m to 350 $\mu$m available under the trade designation AQUA KEEP SA60N TYPE II from Sumitomo Seika Chemicals Col, Ltd. (Japan). Also available super absorbent materials are Luquasorb 1010 and Luquasorb 1030 from BASF, Ludwigshafen, Germany.

Thus in summary, the absorbent(s) utilized in the adhesive compositions of the present subject matter is typically one or more agents selected from (i) insoluble swellable polymers, (ii) hydratable polymers, (iii) water soluble polymers, (iv) synthetic absorbents, (v) super absorbent polymers, and/or (vi) combinations of any one or more of (i)-(v).

For certain embodiments, it is useful to utilize one or more types or grades of carboxymethyl cellulose (CMC) in the present subject matter compositions and methods. CMC is a cellulose ether comprised of repeating cellobiose units. These are composed of two anhydroglucose units (beta-glucopyranose residues). A parameter used in referring to grades of CMC is the degree of polymerization. This is the number of anhydroglucose units which are joined through 1, 4 glucosidic linkages. Each anhydroglucose unit contains three hydroxyl groups. By substituting carboxymethyl groups for some of the hydrogens of the hydroxyl groups, sodium carboxymethyl cellulose is obtained. The average number of hydroxyl groups substituted per anhydroglucose unit is known as the "degree of substitution." If all three hydroxyls are replaced, the maximum theoretical degree of substitution is 3.0 (impossible to practice).

Another parameter used in reference to CMC is average chain length or degree of polymerization. Average chain length (or the degree of polymerization) and the previously noted degree of substitution determine molecular weight of the CMC polymer.

For many embodiments, the CMC utilized in the present subject matter has a degree of substitution of from about 0.2 to about 1.5, and in other embodiments from about 0.7 to about 1.2. In particular embodiments, the degree of substitution of the CMC is from about 0.65 to about 0.90. The molecular weight of CMC is typically within a range of from about 17,000 to about 700,000. The present subject matter includes CMC grades having molecular weights less than 17,000 and greater than 700,000.

In certain versions of the present subject matter, a particularly useful absorbent is sodium carboxymethyl cellulose commercially available from various sources such as from Ashland Chemical under the designation AQUASORB A500. It is also contemplated that instead of, or in addition to, carboxymethyl cellulose; hydroxypropyl cellulose, hydroxypropylmethyl cellulose, and variants thereof can be used in the present subject matter.

Active Agent(s)

Furthermore, a wide array of active agent(s) can be used in the compositions of the present subject matter.

Generally, any active, active agent, or combination of actives and/or active agents which are biologically active and which can be incorporated within the adhesive composition in a stable manner or form, can be utilized. In certain versions of the present subject matter, the active agent is soluble in the vehicle and particularly in polyhydric alcohol(s) when such are utilized as vehicles in the compositions. In certain versions of the present subject matter, the active agent forms a complex with the crystallization inhibitor(s), described in greater detail herein which for example can be polyvinylpyrrolidone. The complex typically results from hydrogen bonding between the active(s) and the inhibitor(s).

In certain aspects, the active(s) can be for example the pain relievers or analgesics fentanyl, butorphanol, morphine, buprenorphine, naloxone, codeine, menthol, methyl salicylate, camphor, capsaicin, acetylsalicylic acid; local anesthetics such as lidocaine; anti-acne drugs like retinoic acid; anti-angina drugs like nitroglycerin, isosorbide dinitrate, nifedipine, nicardipine; antiarrhythmics like timolol; antibacterials like amikacin, cephalosporins, macrolides, tetracyclines, quinolones, nitrofurantoin; anti-convulsives like carbamazepine, phenobarbital, nitrazepam; antidepressants like tricyclics, bupropion, sertraline, pergolide, fluoxetine; anti-rheumatics like diclofenac, ibuprofen, piroxicam, ketoprofen, thiocolchicoside, methotrexate; sex hormones like progesterone, testosterone, estradiol, levonorgestrel; anti-fungals like clotrimazole, ketoconazole, miconazole; anti-hypertensives like sotalol, alprenolol, captopril, enalapril, felodipine, nicardipine, reserpine; anti-hypothyroid drugs like thyroxine; anti-malarials like artemesine, cinchonidine, primaquine; anti-migraine drugs like ergotamine, sumatriptan, rizatriptan; anti-nausea drugs like domperidone, chlorpromazine, methoclopramide, scopolamine, tetrahydrocannabinoids; skin lighteners like hydroquinone, hydroquinine; dopamine receptor antagonists like pergolide, bromocriptine; muscle relaxants like thiocolchicoside, diazepam; sclerosing agents like ethanolamine, sodium ricinoleate; vitamins like A, B, C, E and precursors or various agents like oxybutynin, finasteride, erythropoetine. Combinations of one or more of these actives are also contemplated including combinations of these agents with still other ingredients.

Crystallization Inhibitor(s)

The present subject matter in certain versions, includes the use of one or more crystallization inhibitors in the compositions, and thus crystallization inhibitors are optional. In certain versions of the present subject matter, the adhesive compositions may be free of such inhibitors. Crystallization inhibitors are used to enhance the solubility and stability of the actives in the compositions. Crystallization inhibitors provide for higher concentrations/loading of active agents to be dissolved and once dissolved, inhibit the actives from subsequently precipitating out of solution. By use of these strategies, the crystallization inhibitors thereby increase the availability of the actives for application to human skin during transdermal drug delivery. Although not wishing to be bound to any particular theory, it is believed that, because more soluble actives are available within the adhesive matrix, the crystallization inhibitors indirectly increase the amount of actives delivered to the skin. High concentrations of dissolved active ingredients in the matrix of transdermal therapeutic systems generally make possible a high flow of active ingredients to and through the skin to aid in treatment.

A wide range of crystallization inhibitors can be incorporated in the present subject matter compositions to enhance the availability of the solvated actives for treatment. For example, the crystallization inhibitor may comprise polyvinylpyrrolidone, polyacrylamides, polyvinyl alcohols, polyacrylic acids, caseins, gelatins, polyamines/polyethyleneimines, polyethylene glycols, cellulose, cellulose derivatives, methylcellulose, hydroxypropyl cellulose, ethyl cellulose, carboxymethylcellulose, non-urethane associated thickeners, quaternary ammonium alginates, xanthan, pectin, guar gum, guar gum derivatives, carrageenan, carboxypolymethylene, agar, polyethoxylated sorbitols, butyl methacrylate, butyl methacrylate derivatives, 2-dimethylaminoethyl methacrylate, methyl methacrylate, polyaminoamides, polyaminoimidazolines, polyetherurethaneamines, polyethylene oxide, polyacrylic acid, silica, silicon dioxide, starch, starch derivatives, dextrin, cyclodextrins, dextran, rosin esters, sterols, bile acids, polyglucosamines, monoacylglycerols, glycerol monooleate, glycerol monolinoleate, glycerol monopalmitate, glycerol monostearate, glycerol monolaurate, glycerol monocaprylate, glycerol monocaprate, and combinations or mixtures thereof.

In certain versions of the present subject matter, the crystallization inhibitor includes polyvinylpyrrolidone (PVP), either alone or in combination with other crystallization inhibitors. PVP is a white, hygroscopic polymer with a weak characteristic odor. PVP is usually in powder form, although it can be in solution, and comprises the monomer N-vinylpyrrolidone as the base unit. By selecting suitable polymerization conditions, a wide range of molecular weights can be obtained, extending from low values of a few thousand daltons to approximately 2.2 million daltons, i.e. 2,200 kDa.

PVP can be either a homopolymer or copolymer, typically synthesized by free-radical polymerization in water or alcohols with a suitable initiator of vinylpyrrolidone (also referred to as N-vinylpyrrolidone, N-vinyl-2-pyrrolidone and N-vinyl-2-pyrrolidinone) as a monomeric unit. PVP polymers include soluble and insoluble homopolymeric PVPs, and copolymers such as vinylpyrrolidone/vinyl acetate and vinylpyrrolidone/dimethylamino-ethylmethacrylate. Substantially cross-linked homopolymers of PVP are insoluble and are generally known in the pharmaceutical industry under the designations polyvinylpolypyrrolidone, crospovidone and PVP. The copolymer vinylpyrrolidone-vinyl acetate is generally known in the pharmaceutical industry under the designations Copolyvidon(e), Copolyvidonum or VP-VAc.

In certain aspects of the present subject matter, the PVP is soluble. The term "soluble" when used with reference

EP 4 162 931 A1

to PVP means that the polymer is soluble in water and generally is not substantially cross-linked, and has a weight average molecular weight of less than about 2,200,000. In contrast to most polymers, soluble PVP is readily soluble in water and also in a large number of organic solvents, such as alcohols, amines, acids, chlorinated hydrocarbons, amides and lactams. Soluble PVP polymers have been identified in the pharmaceutical industry under a variety of names, the most commonly used include Povidone, Polyvidon(e), Polyvidonum, Polyvidonum, poly(N-vinyl-2-pyrrolidinone, poly(N-vinylbutyrolactam), poly(1-vinyl-2-pyrrolidone), poly[1-(2-oxo-1-pyrrolidinyl)ethylene]. PVP homopolymer is generally insoluble in the common esters, ethers, hydrocarbons and ketones. When dry, soluble PVP homopolymer is a light flaky powder, which absorbs up to 40% of its weight in water.

The amount and type of PVP required in the embodiments typically depends on the quantity and type of drug present in the adhesive composition, as well as the type of adhesives. Typically, the PVP is present in an amount from about 0.01% to about 30% by weight of the weight of the total adhesive composition. The soluble PVP for certain versions of the present subject matter has a weight average molecular weight of less than about 2,200 kilodaltons (kDa), more particularly less than about 100 kDa, and most particularly less than 54 kDa. In certain versions, it is useful to employ PVP having a weight average molecular weight from about 2,000 to 2,200,000 (i.e. 2 kDa to 2,200 kDa), more particularly from 5,000 to 100,000 (i.e. 5 kDa to 100 kDa), and most particularly 7,000 to 54,000 (i.e. 7 kDa to 54 kDa). In certain versions of the present subject matter it is useful to employ PVP having certain characteristics and/or properties. For example, in certain embodiments, the PVP has a weight average molecular weight (Mw) of from about 9 to about 850 kilodaltons (kDa), and a number average molecular weight (Mn) of from about 2 to about 200 kDa. In certain aspects, the PVP has a glass transition temperature of from about 110 °C to about 180 °C. And, in certain embodiments, the PVP has a K-value of from about 15 to about 82. It is also contemplated to utilize PVP exhibiting all of these characteristics.

As noted, a wide range of crystallization inhibitors can be incorporated in the present subject matter compositions. In certain versions of the present subject matter, the crystallization inhibitor is polyvinylpyrrolidone (PVP). In contrast to most polymers, PVP is readily soluble in water and a large number of organic solvents, such as alcohols, amines, acids, chlorinated hydrocarbons, amides and lactams. The polymer is generally insoluble in the common esters, ethers, hydrocarbons and ketones.

PVP is typically synthesized by free-radical polymerization of N-vinylpyrrolidone in water or alcohols with a suitable initiator. By selecting suitable polymerization conditions, a wide range of molecular weights can be obtained, extending from low values of a few thousand daltons to approximately 2.2 million daltons.

In certain versions of the present subject matter, it is advantageous to utilize one or more commercially available grades of PVP, such as those under the tradename LUVITEC® from BASF Corporation. BASF offers a wide range of vinylpyrrolidone homopolymers with different molecular weights (K-values) under the name LUVITEC® K. The products are available as a powder or as aqueous solutions. Characteristic parameters of LUVITEC® K grades are listed in Table 2.

Table 2 - Representative Grades of PVP

|  | K-value | Solids content in % | pH-value (10% solution) | Residual NVP content in ppm | Brookfield-RVT Viscosity in mPa•s at 23 °C |
|---|---|---|---|---|---|
| LUVITEC® K 17 powder | 15-19 | 95.0-100.0 | 3.0-7.0 | ≤ 100 | 80-180 (40/2/100) |
| LUVITEC® K 30 powder | 27-33 | 95.0-100.0 | 3.0-7.0 | ≤ 100 | 80-140 (30/1/50) |
| LUVITEC® K 80 powder | 74-82 | 95.0-100.0 | 5.0-8.0 | ≤ 100 | 2500-7000 (20/6/100) |
| LUVITEC® K 85 powder | 84-88 | 95.0-100.0 | 5.0-9.0 | ≤ 100 | 5000-20000 (20/6/50) |
| LUVITEC® K 90 powder | 88-92 | 95.0-100.0 | 5.0-9.0 | ≤ 100 | 10000-25000 (20/7/100) |
| LUVITEC® K 90 HM powder | 92-96 | 95.0-100.0 | 5.0-9.0 | ≤ 100 | 15000-30000 (20/7/100) |
| LUVITEC® K 30 solution approx. 30% | 27-33 | 29.0-31.0 | 4.0-8.0 | ≤ 100 | 80-140 (30/1/50) |
| LUVITEC® K 60 solution approx. 35% | 52-62 | 34.0-36.0 | 7.0-9.0 | ≤ 300 | 2000-20000 (35/6/50) |
| LUVITEC® K 85 CQ solution approx. 20% | 83-88 | 19.0-21.0 | 7.0-9.0 | ≤ 100 | 5000-15000 (20/6/50) |
| LUVITEC® K 90 CQ solution approx. 10% | 90-98 | 9.5-10.5 | 7.0-9.0 | ≤ 50 | 300-1500 (10/4/100) |

(continued)

|  | K-value | Solids content in % | pH-value (10% solution) | Residual NVP content in ppm | Brookfield-RVT Viscosity in mPa•s at 23 ºC |
|---|---|---|---|---|---|
| LUVITEC® K 90 solution approx. 20% | 90-98 | 19.0-21.0 | 7.0-9.0 | ≤ 100 | 10000-40000 (20/7/100) |
| LUVITEC® K 115 CQ solution approx. 10% | 114-130 | 10.5-11.5 | 7.0-9.0 | ≤ 50 | 2000-5000 (11/6/100) |

Table 3 set forth below, presents typical properties of various grades of PVP commercially available under the LUVITEC tradename.

Table 3 - Typical Properties of PVP

| LUVITEC® | K 17 | K 30 | K 60 | K 80 | K 85 | K 90 | K 90 HM | K 115 |
|---|---|---|---|---|---|---|---|---|
| Molecular weight (GPC): Mw in kDa | 9 | 50 | 450 | 850 | 1100 | 1400 | 1800 | 2200 |
| Mn in kDa | 2 | 14 | 140 | 200 | 250 | 325 | 375 | 400 |
| Ash content in %* | | | | ≤0.02 | | | | |
| Rel. Viscosity (1% in water, 23 ºC capillary viscometer) | 1.09 (5%: 1.53) | 1.25 | 1.93 | 3.09 | 3.74 | 5.09 | 5.69 | 12.1 (0.1%: 1.33) |
| Glass transition temperature in ºC (DSC) | 110 | 175 | 175 | 180 | 180 | 180 | 180 | 180 |
| Particle size in μm (Sympatec-Helos Rodos) | | | | | | | | |
| $X_{10}$% | 15 | 25 | Only available as solution | 60 | 90 | 90 | 90 | Only available as solution |
| $X_{50}$% | 25 | 75 | | 160 | 180 | 180 | 180 | |
| $X_{90}$% | 100 | 130 | | 320 | 350 | 350 | 350 | |
| Color (10% solution, according to Europ. Pharmacopoeia) | | | Brighter than BY5/B5/R6 | | | | | |
| Moisture absorption at saturation in % | | | 20 (50% rel. humidity, 23 ºC)  40 (75% rel. humidity, 23 ºC) | | | | | |

In certain versions of the present subject matter it is useful to employ PVP having certain characteristics and/or properties. For example, in certain embodiments, the PVP has a weight average molecular weight (Mw) of from about 9 to about 850 kDa, and a number average molecular weight (Mn) of from about 2 to about 200 kDa. In certain aspects, the PVP has a glass transition temperature of from about 110 °C to about 180 °C. And, in certain embodiments, the PVP has a K-value of from about 15 to about 82. It is also contemplated to utilize PVP exhibiting all of these characteristics.

Vehicle(s)

And, a wide array of vehicles, carriers, and/or solvents can be utilized in the present subject matter compositions and thus vehicles are optional. Generally, one or more polyhydric alcohols are utilized in the present subject matter compositions. Suitable examples of polyhydric alcohols include dihydric alcohols, such as ethylene glycols, polyethylene glycols, propylene glycols, 1,3- and 1,4-butanediols, 1,6-hexanediol, diethylene qlycol, bis(hydroxymethyl)cyclohexane, bis(hydroxyethyl)benzene, hydrogenated bisphenol A, hydrogenated bisphenol F, polytetramethylene glycols, polyester diols and silanol-terminated polysiloxanes; trihydric alcohols, such as glycerol, trimethylol propane, trimethylol ethane, 1,2,3-butane triol, 1,2,6-hexane triol and polyester triols; and polyhydric alcohols having 4 to 8 or more hydroxyl groups, such as pentaerythritol, diglycerol, $\alpha$-methylglucoside, sorbitol, xylitol, mannitol, glucose, fructose, sucrose, and the like. However, it will be appreciated that the present subject matter includes the use of other vehicles, carriers, and/or solvents instead of, or in addition to these polyhydric alcohols.

Additional Aspects

In certain embodiments, the adhesive composition is a hydrocolloid adhesive. A base hydrocolloid adhesive formulation generally comprises a hot melt adhesive blended with an absorbent such as sodium carboxymethylcellulose (CMC), gelatin, pectin, alginate, polyacrylate superabsorbent, or the like. Other hydrocarbon resins, such as polyisobutylene, can also be included to adjust adhesive properties. Any of these formulations can be selected for the base adhesive in a drug-delivery application in accordance with the present subject matter.

For effective drug delivery it is advantageous to incorporate a polyhydric alcohol which acts primarily as a vehicle into which the drug is actually dissolved, but may also serve a secondary function as a skin penetration enhancer. Propylene glycol is an example of a polyhydric alcohol which serves both purposes. Other examples of polyhydric alcohol vehicles include glycerol and polyethylene glycols, typically with molecular weights between 200 and 1,000 Da, or any mixtures thereof.

It is further advantageous in certain applications, to include a particular crystallization inhibitor to stabilize the drug or active agent(s). Polyvinylpyrrolidone (PVP) includes soluble PVP homopolymers of low molecular weight. PVP can also increase the viscosity of the polyol phase, in some cases creating a gel, to prevent the polyol phase from migrating and separating from the adhesive matrix.

Once blended together, these ingredients, e.g. certain polyhydric alcohols and certain PVP agents, form a complex multi-phase system in which the various components typically form separate domains but are nevertheless homogeneously blended into a single cohesive mass.

In view of these aspects, the present subject matter provides a hydrocolloid adhesive formulation as set forth below in Table 4. The active agent may for example be ibuprofen.

Table 4 - Representative Adhesive Compositions

| Weight Percentage | Component |
| --- | --- |
| 30% to 80% | Hot Melt Adhesive |
| 0.5% to 45% | Absorbent |
| 0.5% to 10% | Active Agent(s) |
| 0.5% to 20% | Polyvinylpyrrolidone (PVP), (Crystallization Inhibitor) |
| 0.5% to 20% | Polyhydric Alcohol (Vehicle) |

In certain embodiments, the hot melt adhesive includes styrene-isoprene copolymers, the polyhydric alcohol is propylene glycol, the PVP is a soluble homopolymer with molecular weight less than about 60 kDa, and the absorbent is sodium carboxymethyl cellulose (CMC).

Notably, these mixtures can be blended and extruded at temperatures less than 75 °C, which is the critical thermal breakdown temperature of many actives such as ibuprofen. This is a unique feature that cannot be achieved with solvent-based acrylic adhesives because of the high drying temperatures that such adhesives typically require. In certain applications, the adhesives can be prepared by incorporating the various components at temperatures in a range of 60° C to 70° C.

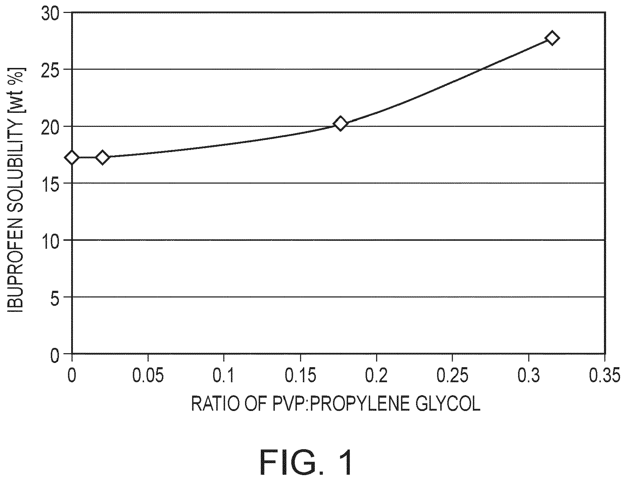

PVP enhances the solubility of certain actives such as ibuprofen in propylene glycol, as shown in Figure 1. Without PVP present, the room temperature saturation concentration is 17.3 wt % ibuprofen in propylene glycol. The solubility of ibuprofen increases to 27.7 wt % when the vehicle is changed to a mixture of 75% propylene glycol and 25% PVP (BASF LUVITEC K17, typical Mw of about 9,000 Da). The enhanced solubility enables increased loading of drug or other active(s) without risk of crystallization.

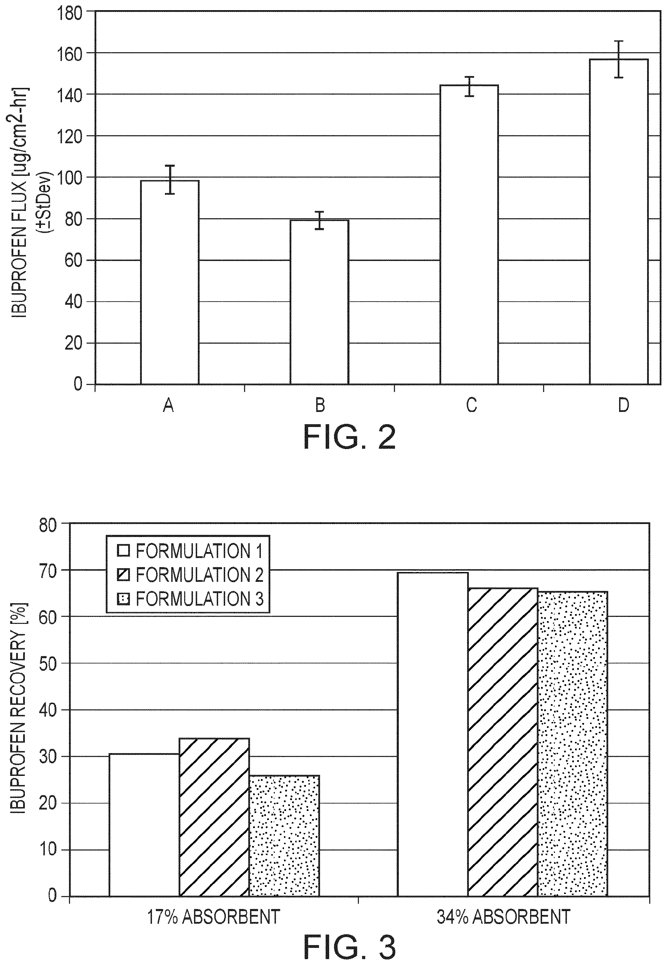

A mixture of propylene glycol and PVP also enhances the release kinetics of ibuprofen, as shown in Figure 2. Compared to a mixture of ibuprofen with hot melt adhesive alone, or compared to a mixture of ibuprofen with hot melt adhesive and absorbent; mixtures incorporating PVP/propylene glycol blends have approximately 50% higher release rates. Specifically, Figure 2 illustrates flux of ibuprofen released from various adhesive formulations measured using Franz diffusion cells employing a silicone rate-limiting membrane and 40% ethanol/saline as the receptor solution. In Figure 2, composition A is 5% ibuprofen in a hot melt adhesive. Composition B is 5% ibuprofen in a hot melt adhesive containing CMC. Composition C is 5% ibuprofen in a hot melt adhesive containing CMC and a mixture of 17% PVP in propylene glycol. And, composition D is 5% ibuprofen in a hot melt adhesive containing CMC and a mixture of 43% PVP in propylene glycol. Compositions C and D represent embodiments of the present subject matter.

Formulations containing PVP are more stable in terms of ibuprofen crystallization when compared to non-PVP-containing counterparts. Differential scanning calorimetry of a 5% ibuprofen formulation containing 10% propylene glycol and 34% CMC reveals a thermal transition that is absent in an equivalent formulation containing PVP. The transition is attributed to the melting of crystals contained with this material that are absent in materials containing PVP.

In accordance with the present subject matter, adding one or more absorbents imparts an unexpected advantage beyond simply allowing the formulation to absorb fluid. Drug release is also enhanced by the inclusion of an absorbent material. In certain versions of the present subject matter, the extent of release of the one or more active agents is increased more than the increase in the amount of the absorbent. For example, in certain embodiments, the extent of release (or rate of release in certain versions) can be increased by 10% upon increasing the amount of absorbent in the adhesive composition by less than 10% such as for example 5%. This aspect may be expressed in terms of multiplication factors. For example, in certain versions of the present subject matter, upon increasing the amount of absorbent in the adhesive composition by a factor of 2.0 (or 200%), the extent of release of an active agent such as ibuprofen is increased by a factor greater than 2.0 (or greater than 200%).

Another particular advantage of the present subject matter compositions, is that the compositions can be processed at low temperatures. This is particularly important for a drug such as ibuprofen, which degrades at 75 ºC. In certain versions of the present subject matter, compositions can be processed at 65-70 ºC. For a solvent-based system, for example, the resulting composition would need to be processed at much higher temperatures in order to dry it or remove vehicle or solvent. Similarly, rubber hot melts would be processed at temperatures of about 150 °C.

In addition to exhibiting the previously noted properties, in particular embodiments of the present subject matter, the adhesive compositions also exhibit a relatively high fluid handling characteristic or ability. The characteristic of relatively high fluid handling ability is exhibited in one or more fashions as follows.

In one aspect, the relatively high fluid handling ability of the present subject matter adhesive compositions is indicated by the compositions exhibiting a static absorption of at least about 50 g/m$^2$/24 hours. In certain versions of the present subject matter, the adhesive compositions exhibit a static absorption of at least 100 g/m$^2$/24 hours; of at least 500 g/m$^2$/24 hours; of at least 1,000 g/m$^2$/24 hours; of at least 2,500 g/m$^2$/24 hours; and in certain embodiments at least 5,000 g/m$^2$/24 hours. In certain versions, the adhesives exhibit a static absorption of from 5,000 to 10,000 g/m$^2$/24 hours.

In another aspect, the relatively high fluid handling characteristics of the adhesive compositions is indicated by its moisture vapor transmission rate (MVTR). Generally, the MVTR of the present subject matter adhesive compositions is at least 25 g/m$^2$/24 hours. In certain embodiments of the present subject matter, the adhesive compositions exhibit MVTR values of at least 50 g/m$^2$/24 hours; at least 100 g/m$^2$/24 hours; at least 200/g/m$^2$/24 hours; at least 350 g/m$^2$/24 hours; and in certain versions, greater than 500 g/m$^2$/24 hours. In certain versions, the adhesive compositions exhibit a MVTR from 500 to 1000 g/m$^2$/24 hours.

In certain versions of the present subject matter, the adhesive compositions exhibit a static absorption of at least 500 g/m$^2$/24 hours and an MVTR value of at least 25 g/m$^2$/24 hours. A description of determining static absorption and MVTR is provided herein.

Methods

The present subject matter also provides various methods. In one version, a method of forming an adhesive composition which releases at least one active agent is provided. The method comprises providing an adhesive component including an adhesive and a vehicle. The adhesive and the vehicle are described herein. The method also comprises incorporating 0.1% to 50% of at least one absorbent, 0.1% to 20% of at least one active agent, and 0% to 30% of at least one crystallization inhibitor into the adhesive component to thereby form an adhesive composition which releases the at least one active agent. The absorbent(s), active agent(s), and inhibitor(s) are as described herein. The various components can be incorporated with one another, blended, and/or otherwise combined in techniques or operations known in the art.

The present subject matter also provides a method of releasing at least one active agent to a region of interest such as for example on biological skin. The method comprises providing an article adapted for placement along biological skin. The article generally defines at least one face. The method also comprises providing an adhesive composition including an adhesive component, a vehicle, 0.1% to 50% of at least one absorbent, 0.1% to 20% of at least one active agent, and 0% to 30% of at least one crystallization inhibitor. The method further comprises depositing the adhesive composition onto the face of the article. And, the method comprises applying the article to a region of interest on biological skin, whereby the adhesive composition is directed toward and overlies the region of interest. The active agent(s) is then released from the adhesive composition and is transferred to the region of interest such as a wound or other region on biological skin. The components of the adhesive composition are as described herein.

In certain embodiments, the adhesive compositions of the present subject matter exhibit particular Peel on polyethylene values and/or particular Tack values. The measurement of these properties is described in greater detail herein in conjunction with various evaluations described herein. In certain versions, the adhesive compositions exhibit a Peel on polyethylene value of at least 0.5 N/in, more particularly at least 1.5 N/in, and more particularly at least 2.5 N/in. In certain versions, the adhesive compositions exhibit a Tack value of at least 10 N/in, more particularly at least 25 N/in, and more particularly at least 40 N/in.

Medical Articles

The adhesive compositions described herein can be used in association with a wide array of medical articles. Nonlimiting examples of such articles include wound dressings, surgical dressings, medical tapes, athletic tapes, surgical tapes, sensors, electrodes, ostomy appliances or related components such as sealing rings, catheters, connector fittings, catheter hubs, catheter adapters, fluid delivery tubes, electrical wires and cables, negative pressure wound therapy (NPWT) components, surgical drains, wound draining components, IV site dressings, prostheses, stoma pouches, buccal patches, transdermal patches, dentures, hairpieces, bandages, diapers, medical padding for example liposuction padding, hygiene pads, corn and callous pads, blister cushioning and protecting pads, toe cushioning pads, and pads for protecting and cushioning tube sites such as tracheotomy tubes. The medical articles include one or more regions or surfaces to which the adhesive compositions of the present subject matter are applied. Forming a layer, coating, or other region of adhesive on an article enables the article to be adhered to a wide range of surfaces, including skin. It will be understood that the present subject matter is not limited to any of these articles. Instead, the subject matter includes the use of the adhesive compositions with other articles besides those noted herein. The medical articles may also include one or more layers covering the adhesive layer or coating such as a release liner.

Examples

Evaluations were undertaken to assess several adhesive compositions in accordance with the present subject matter. The materials used in the adhesive compositions were as follows.

Adhesive: T2650 (Avery Dennison), styrene-isoprene hot melt adhesive;

Absorbent: AQUASORB A500 (Ashland), sodium carboxymethyl cellulose;

PVP: LUVITEC K17 (BASF), soluble PVP homopolymer with average molecular weight of 9 kDa;

Drug: Ibuprofen (BASF), USP-grade;

Vehicle: propylene glycol;

Three different adhesive formulations were prepared as follows:

5% ibuprofen, 10% propylene glycol, 2% PVP (Formulation 1)

5% ibuprofen, 10% propylene glycol, 5% PVP (Formulation 2)

5% ibuprofen, 10% propylene glycol, 7.5% PVP (Formulation 3)

In a first set of trials, 17% absorbent was added (the remainder of the mixture being adhesive), and in a second set 34% absorbent was added. Samples were soaked in a mixture of ethanol and water for two hours and then the solvent was sampled to measure the total amount of ibuprofen extracted from the mixture. In all three cases, including 34% absorbent resulted in more than twice as much ibuprofen recovery, as shown in Figure 3. Specifically, as shown in Figure 3, the ibuprofen recovery for formulations 1 and 3 containing 17% absorbent was about 30% and 26%, respectively; and for the same formulations containing 34% absorbent, the ibuprofen recovery was increased to almost 70% and about 66%, respectively. These gains were greater than the relative increase in the proportion of absorbent, i.e. from 17% to 34%.

Thus, in certain aspects, the present subject matter provides methods of increasing the extent of release of one or more actives in an adhesive composition by increasing the amount or proportion of absorbent in the composition. In certain versions, increasing the amount of absorbent in the adhesive composition by a factor of 2.0 results in the extent of release of ibuprofen being increased by a factor greater than 2.0. Moreover, increasing the amount or proportion of absorbent also increases the rate and extent of ibuprofen release as detailed in the following evaluation.

In another evaluation, the effect of absorbent on ibuprofen release was investigated. In this evaluation, a series of formulations were prepared using T2560 (hot melt adhesive), AQUASORB A500 (sodium carboxymethyl cellulose absorbent), propylene glycol, and various grades of polyvinylpyrrolidone (LUVITEC K80, LUVITEC K30, and LUVITEC K17 from BASF). 5% ibuprofen was included in each formulation.

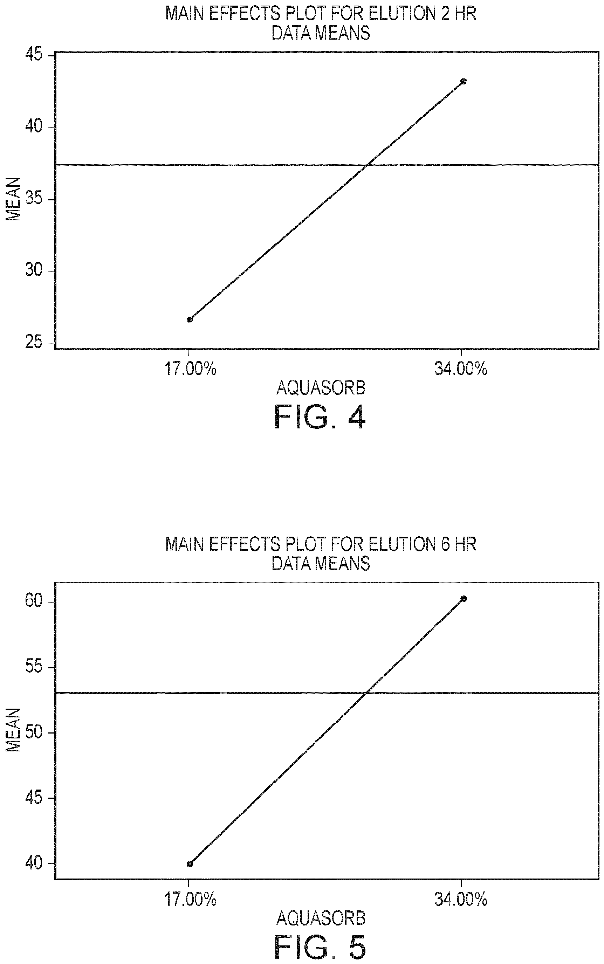

Ibuprofen elution was measured by soaking an amount or "patch" of adhesive in a mixture of 40% ethanol in saline (0.8% NaCl in water), and sampling the liquid phase after 2 hrs and again after 6 hrs. The concentration of ibuprofen in the solution was measured by HPLC and this value was used to calculate the ibuprofen released from the patch as a percentage of the total ibuprofen contained in the patch. The formulation list and results are set forth in Table 5 below. A Main Effects analysis using MINITAB® statistical software from Minitab Inc. was undertaken which revealed that higher amounts of A500 led to greater ibuprofen release. Referring to Figures 4 and 5, on average, doubling the amount of A500 from 17% to 34% led to a substantial increase in ibuprofen elution after both 2 and 6 hours of soak time.

Table 5 - Representative Formulations and Elution Results

| Formulation ID | T2560 Hot Melt Adhesive | A500 Absorbent | Propylene Glycol | LUVITEC K80 PVP | LUVITEC K30 PVP | LUVITEC K17 PVP | Ibuprofen | Elution 2 hrs [% of total ibu] | Elution 6 hrs [% of total ibu] |
|---|---|---|---|---|---|---|---|---|---|
| 1 | 66.00% | 17.00% | 10.0% | 2.0% | 0.00% | 0.00% | 5.00% | 20 | 30 |
| 2 | 49.00% | 34.00% | 10.0% | 2.0% | 0.00% | 0.00% | 5.00% | 56 | 79 |
| 3 | 63.00% | 17.00% | 10.0% | 5.0% | 0.00% | 0.00% | 5.00% | 32 | 29 |
| 4 | 46.00% | 34.00% | 10.0% | 5.0% | 0.00% | 0.00% | 5.00% | 31 | 59 |
| 5 | 60.50% | 17.00% | 10.0% | 7.5% | 0.00% | 0.00% | 5.00% | 17 | 25 |
| 6 | 43.50% | 34.00% | 10.0% | 7.5% | 0.00% | 0.00% | 5.00% | 20 | 39 |
| 7 | 66.00% | 17.00% | 10.0% | 0.0% | 0.00% | 2.00% | 5.00% | 31 | 52 |
| 8 | 49.00% | 34.00% | 10.0% | 0.0% | 0.00% | 2.00% | 5.00% | 69 | 69 |
| 9 | 63.00% | 17.00% | 10.0% | 0.0% | 0.00% | 5.00% | 5.00% | 34 | 63 |
| 10 | 46.00% | 34.00% | 10.0% | 0.0% | 0.00% | 5.00% | 5.00% | 66 | 75 |
| 11 | 60.50% | 17.00% | 10.0% | 0.0% | 0.00% | 7.50% | 5.00% | 26 | 42 |
| 12 | 43.50% | 34.00% | 10.0% | 0.0% | 0.00% | 7.50% | 5.00% | 66 | 71 |
| 13 | 61.00% | 34.00% | 0.0% | 0.0% | 0.00% | 0.00% | 5.00% | 33 | 50 |
| 14 | 51.00% | 34.00% | 10.0% | 0.0% | 0.00% | 0.00% | 5.00% | 37 | 55 |
| 15 | 49.00% | 34.00% | 10.0% | 0.0% | 2.00% | 0.00% | 5.00% | 37 | 62 |
| 16 | 46.00% | 34.00% | 10.0% | 0.0% | 5.00% | 0.00% | 5.00% | 32 | 56 |
| 17 | 43.50% | 34.00% | 10.0% | 0.0% | 7.50% | 0.00% | 5.00% | 30 | 50 |

In another evaluation, various compositions were prepared. For each composition, a comparative composition was prepared.

For each resulting set of compositions, the following measurements were made: (i) adhesion to polyethylene, i.e. "Peel", (ii) Tack, (iii) release of active after 2 hours, i.e. "Elution 2 hr", (iv) release of active after 6 hours, i.e. "Elution 6 hr", and (v) fluid handling capacity, i.e. "FHC".

Specifically, samples were prepared and evaluated as follows.

Sample Preparation

Adhesive components were blended together in a sigma-blade mixer heated to approximately 70°C. After blending, all adhesive specimens were pressed into sheets approximately 0.8 mm thick and laminated on one side to an adhesive-coated polyurethane film, 70 microns in thickness.

Peel

Specimens measuring one inch wide by approximately five inches long were applied to the surface of a polyethylene film using a 4.5 lb roller and allowed to dwell for approximately one minute before peeling off at a ninety degree angle and a rate of 12 inches/minute using a tensile tester to record the average force required for removal.

Tack

Specimens were mounted to a fixture, adhesive side up. A piece of 5 mil polyester film measuring 15 cm long by 2.54 cm wide was formed into a loop and brought into contact with the adhesive surface, making a 1 inch $\times$ 1 inch contact area. Then the polyester loop was pulled away from the adhesive. A tensile tester was used to contact and pull the loop away from the adhesive at a crosshead speed of 12 inches/min, and the peak force measured during removal was taken as the quantitative measurement of tack.

Elution

A disk was cut from each specimen measuring 0.75 inches in diameter and placed in a vial containing approximately 20 mL of a solution of ethanol and saline (40% ethanol by volume in a solution of 0.9% sodium chloride in water by weight). The vial was tumbled in ambient temperature conditions, and after two and six hours samples of the solution were taken out for analysis. The concentration of ibuprofen in the solution was quantified using ultraviolet absorbance allowing the percentage ibuprofen release to be calculated as a percentage by weight of the total amount present in the sample.

Fluid Handling Capacity

Fluid Handling Capacity is a measure of the combined ability of the composite to take up moisture and to evaporate it to the environment. This test is performed by laminating a sample cut to the size of a Paddington cup to the cup on the side having the rubber ring. The circular sealing ring is placed on the sample of the cup and the screws are secured. The cup is weighed (W1). The cup is then turned upside down and filled with 20 ml of a saline solution (0.9% wt NaCl and 0.04% wt $CaCl_2$ in deionized water). The metal sealing place is secured to the top side of the cup. The filled cup is weighed (W2). The cup is placed sample side down into an oven at 37° C. for 24 hours. After 24 hours, the cup is removed from the oven and allowed to cool to room temperature for 30 minutes. The cup is then weighed (W3). The metal sealing plate is removed and the cup is emptied. The cup is allowed to stand for 15 minutes on a tissue to remove the saline solution, and then weighed (W4). The test conditions are 23° C. ($\pm 2°$) and 50% ($\pm 2\%$) relative humidity. The samples tested herein had a thickness of 0.8 mm. The Moisture Vapor Transmission Rate (MVTR) equals (W2-W3)$\times$1000. The Static Absorption equal (W4-W1)$\times$1000. The Fluid Handling Capacity (FHC) in g/10 cm2/24 hours is determined as follows:

$$FHC=(W2-W3)+(W4-W1)$$

Tables 6, 8, 10, and 12 present Comparative Composition A-D, respectively, lacking absorbent. These tables also present compositions in accordance with the present subject matter in which absorbent is included, i.e. Compositions 1-4. Tables 7, 9, 11, and 13 present the results of testing in which Peel, Tack, elution, and fluid handling capacity were evaluated.

Table 6 - Formulations

|  | Composition 1 | Comparative Composition A |
|---|---|---|
|  | Parts [Weight Basis] | Parts [Weight Basis] |
| Hot Melt Adhesive | 100 | 100 |
| Propylene Glycol | 4.4 | 4.3 |
| Polyvinylpyrrolidone | 2.9 | 2.7 |
| Ibuprofen | 7.2 | 6.9 |
| Absorbent | 28 | 0 |

Table 7 - Results of Testing

|  | Composition 1 | Comparative Composition A |
|---|---|---|
| Peel [N/in] | 2.8 | 0.5 |
| Tack [N/in] | 50 | 40 |
| Elution 2 hr [%] | 20 | 12 |
| Elution 6 hr [%] | 35 | 23 |
| FHC [$g/m^2$-day] | 6,300 | 200 |

Table 8 - Formulations

|  | Composition 2 | Comparative Composition B |
|---|---|---|
|  | Parts [Weight Basis] | Parts [Weight Basis] |
| Hot Melt Adhesive | 100 | 100 |
| Propylene Glycol | 4.3 | 4.5 |
| Polyvinylpyrrolidone | 0 | 0 |
| Ibuprofen | 7.2 | 7.4 |
| Absorbent | 29 | 0 |

Table 9 - Results of Testing

|  | Composition 2 | Comparative Composition B |
|---|---|---|
| Peel [N/in] | 0.0 | 0.7 |
| Tack [N/in] | 10 | 35 |
| Elution 2 hr [%] | 22 | 15 |
| Elution 6 hr [%] | 44 | 28 |
| FHC [$g/m^2$-day] | 7,500 | 200 |

Table 10 - Formulations

|  | Composition 3 | Comparative Composition C |
|---|---|---|
|  | Parts [Weight Basis] | Parts [Weight Basis] |
| Hot Melt Adhesive | 100 | 100 |

(continued)

|  | Composition 3 | Comparative Composition C |
|---|---|---|
|  | Parts [Weight Basis] | Parts [Weight Basis] |
| Propylene Glycol | 6.9 | 7.3 |
| Polyvinylpyrrolidone | 8.9 | 9.4 |
| Ibuprofen | 8.3 | 8.7 |
| Absorbent | 38 | 0 |

Table 11 - Results of Testing

|  | Composition 3 | Comparative Composition C |
|---|---|---|
| Peel [N/in] | 2.6 | 0.3 |
| Tack [N/in] | 41 | 42 |
| Elution 2 hr [%] | 16 | 9 |
| Elution 6 hr [%] | 31 | 19 |
| FHC [g/m$^2$-day] | 8,400 | 300 |

Table 12 - Formulations

|  | Composition 4 | Comparative Composition D |
|---|---|---|
|  | Parts [Weight Basis] | Parts [Weight Basis] |
| Hot Melt Adhesive | 100 | 100 |
| Propylene Glycol | 7.0 | 7.0 |
| Polyvinylpyrrolidone | 0 | 0 |
| Ibuprofen | 8.2 | 8.3 |
| Absorbent | 38 | 0 |

Table 13 - Results of Testing

|  | Composition 4 | Comparative Composition D |
|---|---|---|
| Peel [N/in] | 0.0 | 0.1 |
| Tack [N/in] | 8 | 38 |
| Elution 2 hr [%] | 25 | 15 |
| Elution 6 hr [%] | 52 | 28 |
| FHC [g/m$^2$-day] | 8,300 | 200 |

As evident from Tables 6-13, incorporating absorbent into an adhesive formulation significantly increases elution of ibuprofen (both after 2 hours and after 6 hours). Specifically, as shown in Tables 6 and 7, incorporating absorbent into an adhesive composition resulted in an increase in elution at 2 hours from 12% to 20%, and an increase in elution at 6 hours from 23% to 35%. As shown in Tables 8 and 9, incorporating absorbent into an adhesive composition resulted in an increase in elution at 2 hours from 15% to 22%, and an increase in elution at 6 hours from 28% to 44%. As shown in Tables 10 and 11, incorporating absorbent into an adhesive composition resulted in an increase in elution at 2 hours from 9% to 16%, and an increase in elution at 6 hours from 19% to 31%. And, as shown in Tables 12 and 13, incorporating absorbent into an adhesive composition resulted in an increase in elution at 2 hours from 15% to 25%, and an increase in elution at 6 hours from 28% to 52%.

Many other benefits will no doubt become apparent from future application and development of this technology.

All patents, applications, standards, and articles noted herein are hereby incorporated by reference in their entirety.

As described hereinabove, the present subject matter solves many problems associated with previous strategies, systems or devices. However, it will be appreciated that various changes in the details, materials and arrangements of components and operations, which have been herein described and illustrated in order to explain the nature of the subject matter, may be made by those skilled in the art without departing from the principle and scope of the subject matter, as expressed in the appended claims.

Further, the present invention relates to the following items 1 to 131:

1. A method of releasing at least one active agent to a region of interest on biological skin, the method comprising:

providing an article adapted for placement along biological skin, the article defining at least one face;
providing an adhesive composition including an adhesive component, 0.1% to 50% of at least one absorbent, and 0.1% to 20% of at least one active agent;
depositing the adhesive composition onto the face of the article;
applying the article to a region of interest on biological skin, whereby the adhesive composition is directed toward and overlies the region of interest.

2. The method of item 1 wherein the adhesive composition contacts at least a portion of the region of interest on biological skin.

3. The method of any one of items 1-2 wherein the absorbent is selected from the group consisting of (i) insoluble swellable polymers, (ii) hydratable polymers, (iii) water soluble polymers, (iv) synthetic absorbents, (v) super absorbent polymers, and (vi) combinations of any one or more of (i) - (v).

4. The method of any one of items 1-2 wherein the absorbent is carboxymethyl cellulose.

5. The method of item 4 wherein the carboxymethyl cellulose has a degree of substitution within a range from 0.2 to 1.5.

6. The method of any one of items 4-5 wherein the carboxymethyl cellulose has a molecular weight in a range from 17,000 to 700,000.

7. The method of any one of items 1-6 wherein the at least one active agent is selected from the group consisting of analgesics, local anesthetics, anti-acne agents, anti-angina agents, antiarrhythmics, antibacterial, anti-convulsives, antidepressants, anti-rheumatics, sex hormones, anti-fungals, anti-hypertensives, anti-hypothyroid agents, anti-malarials, anti-migraine agents, anti-nausea agents, skin lighteners, dopamine receptor antagonists, muscle relaxants, sclerosing agents, vitamins and combinations thereof.

8. The method of any one of items 1-7 wherein the at least one active agent is an anti-rheumatic.

9. The method of item 8 wherein the anti-rheumatic is ibuprofen.

10. The method of any one of items 1-9 wherein the adhesive composition further includes a vehicle.

11. The method of item 10 wherein the vehicle is a polyhydric alcohol.

12. The method of item 11 wherein the polyhydric alcohol is selected from the group consisting of propylene glycol, glycerol, polyethylene glycol, and combinations thereof.

13. The method of item 12 wherein the polyhydric alcohol is propylene glycol.

14. The method of any one of items 1-13 wherein the adhesive composition further includes at least one crystallization inhibitor.

15. The method of item 14 wherein the crystallization inhibitor includes polyvinylpyrrolidone.

16. The method of any one of items 14-15, wherein the crystallization inhibitor is present in the adhesive composition at a concentration from 0.1% to 30%.

17. The method of any one of items 1-16 wherein the adhesive composition exhibits a Peel on polyethylene value of at least 0.5 N/in.

18. The method of item 17 wherein the adhesive composition exhibits a Peel on polyethylene value of at least 1.5 N/in.

19. The method of item 18 wherein the adhesive composition exhibits a Peel on polyethylene value of at least 2.5 N/in.

20. The method of any one of items 1-19 wherein the adhesive composition exhibits a Tack value of at least 10 N/in.

21. The method of item 20 wherein the adhesive composition exhibits a Tack value of at least 25 N/in.

22. The method of item 21 wherein the adhesive composition exhibits a Tack value of at least 40 N/in.

23. The method of any one of items 1-22 wherein the adhesive composition exhibits a static absorption of at least about 50 $g/m^2/24$ hours.

24. The method of any one of items 1-23 wherein the adhesive composition exhibits a moisture vapor transmission rate (MVTR) of at least 25 $g/m^2/24$ hours.

25. The method of any one of items 1-24 wherein the adhesive composition exhibits a static absorption of at least 500 $g/m^2/24$ hours and a moisture vapor transmission rate (MVTR) of at least 25 $g/m^2/24$ hours.

26. A method of increasing at least one of (i) extent of release and (ii) rate of release of at least one active agent from an adhesive composition including an adhesive component and at least one active agent, the method comprising:
incorporating at least one absorbent in the adhesive composition, whereby the extent and/or rate of release of the at least one active agent is increased.

27. The method of item 26 wherein the active agent is ibuprofen.

28. The method of item 27 wherein the extent of release of the at least one active agent is increased more than the increase in the amount of the absorbent in the adhesive composition.

29. The method of any one of items 27-28 wherein upon increasing the amount of absorbent in the adhesive composition by a factor of 2.0, the extent of release of ibuprofen is increased by a factor greater than 2.0.

30. The method of any one of items 26-29 wherein the adhesive composition further includes a vehicle.

31. The method of any one of items 26-30 wherein the adhesive composition further includes at least one crystallization inhibitor.

32. The method of item 31 wherein the crystallization inhibitor is present in the adhesive composition at a concentration from 0.1% to 30%.

33. The method of any one of items 26-32 wherein the adhesive composition exhibits a Peel on polyethylene value of at least 0.5 N/in.

34. The method of item 33 wherein the adhesive composition exhibits a Peel on polyethylene value of at least 1.5 N/in.

35. The method of item 34 wherein the adhesive composition exhibits a Peel on polyethylene value of at least 2.5 N/in.

36. The method of any one of items 26-35 wherein the adhesive composition exhibits a Tack value of at least 10 N/in.

37. The method of item 36 wherein the adhesive composition exhibits a Tack value of at least 25 N/in.

38. The method of item 37 wherein the adhesive composition exhibits a Tack value of at least 40 N/in.

39. An adhesive composition comprising:

20% to 90% of an adhesive;
0.1% to 50% of at least one absorbent;
0.1% to 20% of at least one active agent(s);
0.1% to 30% of at least one crystallization inhibitor;
0.1% to 30% of a vehicle.

40. The adhesive composition of item 39 wherein the adhesive is a hot melt adhesive.

41. The adhesive composition of item 40 wherein the concentration of the hot melt adhesive is 30% to 80%.

42. The adhesive composition of any one of items 39-41 wherein the absorbent is selected from the group consisting of (i) insoluble swellable polymers, (ii) hydratable polymers, (iii) water soluble polymers, (iv) synthetic absorbents, (v) super absorbent polymers, and (vi) combinations of any one or more of (i) - (v).

43. The adhesive composition of any one of items 39-41 wherein the absorbent is carboxymethyl cellulose.

44. The adhesive composition of item 43 wherein the carboxymethyl cellulose has a degree of substitution within a range from 0.2 to 1.5.

45. The adhesive composition of any one of items 43-44 wherein the carboxymethyl cellulose has a molecular weight in a range from 17,000 to 700,000.

46. The adhesive composition of any one of items 39-45 wherein the concentration of the absorbent is 0.5% to 45%.

47. The adhesive composition of any one of items 39-46 wherein the active agent(s) is selected from the group consisting of analgesics, local anesthetics, anti-acne agents, anti-angina agents, antiarrhythmics, antibacterials, anti-convulsives, antidepressants, anti-rheumatics, sex hormones, anti-fungals, anti-hypertensives, anti-hypothyroid agents, anti-malarials, anti-migraine agents, anti-nausea agents, skin lighteners, dopamine receptor antagonists, muscle relaxants, sclerosing agents, vitamins and combinations thereof.

48. The adhesive composition of item 47 wherein the concentration of the active agent(s) is 0.5% to 10%.

49. The adhesive composition of any one of items 39-48 wherein the crystallization inhibitor is polyvinylpyrrolidone (PVP).

50. The adhesive composition of item 49 wherein the concentration of the polyvinylpyrrolidone is 0.5% to 20%.

51. The adhesive composition of any one of items 39-50 wherein the vehicle includes a polyhydric alcohol.

52. The adhesive composition of item 51 wherein the polyhydric alcohol is selected from the group consisting of propylene glycols, glycerols, polyethylene glycols, and combinations thereof.

53. The adhesive composition of any one of items 51-52 wherein the concentration of the polyhydric alcohol is from 0.5% to 20%.

54. The adhesive composition of any one of items 39-53 wherein the adhesive composition exhibits a Peel on polyethylene values of at least 0.5 N/in.

55. The adhesive composition of item 54 wherein the adhesive composition exhibits a Peel on polyethylene value of at least 1.5 N/in.

56. The adhesive composition of item 55 wherein the adhesive composition exhibits a Peel on polyethylene value of at least 2.5 N/in.

57. The adhesive composition of any one of items 39-56 wherein the adhesive composition exhibits a Tack value of at least 10 N/in.

58. The adhesive composition of item 57 wherein the adhesive composition exhibits a Tack value of at least 25 N/in.

59. The adhesive composition of item 58 wherein the adhesive composition exhibits a Tack value of at least 40 N/in.

60. The adhesive composition of any one of items 39-59 wherein the adhesive composition exhibits a static absorption of at least about 50 g/m$^2$/24 hours.

61. The adhesive composition of any one of items 39-60 wherein the adhesive composition exhibits a moisture vapor transmission rate (MVTR) of at least 25 g/m$^2$/24 hours.

62. The adhesive composition of any one of items 39-61 wherein the adhesive composition exhibits a static absorption of at least 500 g/m$^2$/24 hours and a moisture vapor transmission rate (MVTR) of at least 25 g/m$^2$/24 hours.

63. An adhesive composition comprising:

   30% to 80% of a hot melt adhesive;
   0.5% to 45% of at least one absorbent;
   0.5% to 10% of at least one active agent;
   0.5% to 20% of at least one crystallization inhibitor;
   0.5% to 20% of at least one polyhydric alcohol.

64. The adhesive composition of item 63 wherein the absorbent is selected from the group consisting of (i) insoluble swellable polymers, (ii) hydratable polymers, (iii) water soluble polymers, (iv) synthetic absorbents, (v) super absorbent polymers, and (vi) combinations of any one or more of (i) - (v).

65. The adhesive composition of item 63 wherein the absorbent is carboxymethyl cellulose.

66. The adhesive composition of item 65 wherein the carboxymethyl cellulose has a degree of substitution within a range from 0.2 to 1.5.

67. The adhesive composition of any one of items 65-66 wherein the carboxymethyl cellulose has a molecular weight in a range from 17,000 to 700,000.

68. The adhesive composition of any one of items 63-67 wherein the active agent(s) is selected from the group consisting of analgesics, local anesthetics, anti-acne agents, anti-angina agents, antiarrhythmics, antibacterial, anti-convulsives, antidepressants, anti-rheumatics, sex hormones, anti-fungals, anti-hypertensives, anti-hypothyroid agents, anti-malarials, anti-migraine agents, anti-nausea agents, skin lighteners, dopamine receptor antagonists, muscle relaxants, sclerosing agents, vitamins and combinations thereof.

69. The adhesive composition of any of any one of items 63-68 wherein the polyhydric alcohol is selected from the group consisting of propylene glycols, glycerols, polyethylene glycols, and combinations thereof.

70. The adhesive composition of any one of items 63-69 wherein the adhesive composition exhibits a Peel on polyethylene value of at least 0.5 N/in.

71. The adhesive composition of item 70 wherein the adhesive composition exhibits a Peel on polyethylene value of at least 1.5 N/in.

72. The adhesive composition of item 71 wherein the adhesive composition exhibits a Peel on polyethylene value of at least 2.5 N/in.

73. The adhesive composition of any one of items 63-72 wherein the adhesive composition exhibits a Tack value of at least 10 N/in.

74. The adhesive composition of item 73 wherein the adhesive composition exhibits a Tack value of at least 25 N/in.

75. The adhesive composition of item 74 wherein the adhesive composition exhibits a Tack value of at least 40 N/in.

76. An article adapted to be adhered to biological skin and release at least one active to the skin, the article defining a surface and comprising an adhesive composition disposed on at least a portion of the surface, the adhesive composition comprising:

20% to 90% of an adhesive;
0.1% to 50% of at least one absorbent;
0.1% to 20% of at least one active agent(s);
0.1% to 30% of at least one crystallization inhibitor;
0.1% to 30% of a vehicle.

77. The article of item 76 wherein the adhesive is a hot melt adhesive.

78. The article of item 77 wherein the concentration of the hot melt adhesive is 30% to 80%.

79. The article of any one of items 76-78 wherein the absorbent is selected from the group consisting of (i) insoluble swellable polymers, (ii) hydratable polymers, (iii) water soluble polymers, (iv) synthetic absorbents, (v) super absorbent polymers, and (vi) combinations of any one or more of (i) - (v).

80. The article of any one of items 76-78 wherein the absorbent is carboxymethyl cellulose.

81. The article of item 80 wherein the carboxymethyl cellulose has a degree of substitution within a range from 0.2 to 1.5.

82. The article of any one of items 80-81 wherein the carboxymethyl cellulose has a molecular weight in a range from 17,000 to 700,000.

83. The article of any one of items 76-82 wherein the concentration of the absorbent is 0.5% to 45%.

84. The article of any one of items 76-83 wherein the active agent(s) is selected from the group consisting of analgesics, local anesthetics, anti-acne agents, anti-angina agents, antiarrhythmics, antibacterials, anti-convulsives, antidepressants, anti-rheumatics, sex hormones, anti-fungals, anti-hypertensives, anti-hypothyroid agents, anti-malarials, anti-migraine agents, anti-nausea agents, skin lighteners, dopamine receptor antagonists, muscle relaxants, sclerosing agents, vitamins and combinations thereof.

85. The article of any one of items 76-84 wherein the concentration of the active agent(s) is 0.5% to 10%.

86. The article of any one of items 76-85 wherein the crystallization inhibitor is polyvinylpyrrolidone (PVP).

87. The article of item 86 wherein the concentration of the polyvinylpyrrolidone is 0.5% to 20%.

88. The article of any one of items 76-87 wherein the vehicle includes a polyhydric alcohol.

89. The article of item 88 wherein the polyhydric alcohol is selected from the group consisting of propylene glycols, glycerols, polyethylene glycols, and combinations thereof.

90. The article of any one of items 88-89 wherein the concentration of the polyhydric alcohol is from 0.5% to 20%.

91. The article of any one of items 76-90 wherein the adhesive composition exhibits a Peel on polyethylene value of at least 0.5 N/in.

92. The article of item 91 wherein the adhesive composition exhibits a Peel on polyethylene value of at least 1.5 N/in.

93. The article of item 92 wherein the adhesive composition exhibits a Peel on polyethylene value of at least 2.5 N/in.

94. The article of any one of items 76-93 wherein the adhesive composition exhibits a Tack value of at least 10 N/in.

95. The article of item 94 wherein the adhesive composition exhibits a Tack value of at least 25 N/in.

96. The article of item 95 wherein the adhesive composition exhibits a Tack value of at least 40 N/in.

97. The article of any one of items 76-96 wherein the adhesive composition exhibits a static absorption of at least about 50 g/m$^2$/24 hours.

98. The article of any one of items 76-98 wherein the adhesive composition exhibits a moisture vapor transmission rate (MVTR) of at least 25 g/m$^2$/24 hours.

99. The article of any one of items 76-99 wherein the adhesive composition exhibits a static absorption of at least 500 g/m$^2$/24 hours and a moisture vapor transmission rate (MVTR) of at least 25 g/m$^2$/24 hours.

100. An article adapted to be adhered to biological skin and release at least one active to the skin, the article defining a surface and comprising an adhesive composition disposed on at least a portion of the surface, the adhesive composition comprising:

30% to 80% of a hot melt adhesive;
0.5% to 45% of at least one absorbent;
0.5% to 10% of at least one active agent;
0.5% to 20% of at least one crystallization inhibitor;
0.5% to 20% of at least one polyhydric alcohol.

101. The article of item 100 wherein the absorbent is selected from the group consisting of (i) insoluble swellable polymers, (ii) hydratable polymers, (iii) water soluble polymers, (iv) synthetic absorbents, (v) super absorbent polymers, and (vi) combinations of any one or more of (i) - (v).

102. The article of item 100 wherein the absorbent is carboxymethyl cellulose.

103. The article of item 102 wherein the carboxymethyl cellulose has a degree of substitution within a range from 0.2 to 1.5.

104. The article of any one of items 102-103 wherein the carboxymethyl cellulose has a molecular weight in a range from 17,000 to 700,000.

105. The article of any one of items 100-104 wherein the active agent(s) is selected from the group consisting of analgesics, local anesthetics, anti-acne agents, anti-angina agents, antiarrhythmics, antibacterials, anti-convulsives, antidepressants, anti-rheumatics, sex hormones, anti-fungals, anti-hypertensives, anti-hypothyroid agents, anti-malarials, anti-migraine agents, anti-nausea agents, skin lighteners, dopamine receptor antagonists, muscle relaxants, sclerosing agents, vitamins and combinations thereof.

106. The article of any one of items 100-105 wherein the polyhydric alcohol is selected from the group consisting of propylene glycols, glycerols, polyethylene glycols, and combinations thereof.

107. The article of any one of items 100-106 wherein the adhesive composition exhibits a Peel on polyethylene value of at least 0.5 N/in.

108. The article of item 107 wherein the adhesive composition exhibits a Peel on polyethylene value of at least 1.5 N/in.

109. The article of item 108 wherein the adhesive composition exhibits a Peel on polyethylene value of at least 2.5 N/in.

110. The article of any one of items 100-109 wherein the adhesive composition exhibits a Tack value of at least 10 N/in.

111. The article of item 110 wherein the adhesive composition exhibits a Tack value of at least 25 N/in.

112. The article of item 111 wherein the adhesive composition exhibits a Tack value of at least 40 N/in.

113. A method of forming an adhesive composition which releases at least one active agent, the method comprising:

providing an adhesive component including an adhesive and a vehicle;
incorporating 0.1% to 50% of at least one absorbent, 0.1% to 20% of at least one active agent, and 0.1% to 30% of at least one crystallization inhibitor into the adhesive component to thereby form an adhesive composition which releases the at least one active agent.

114. The method of item 113 wherein the absorbent is selected from the group consisting of (i) insoluble swellable polymers, (ii) hydratable polymers, (iii) water soluble polymers, (iv) synthetic absorbents, (v) super absorbent polymers, and (vi) combinations of any one or more of (i) - (v).

115. The method of item 113 wherein the absorbent is carboxymethyl cellulose.

116. The method of item 115 wherein the carboxymethyl cellulose has a degree of substitution within a range from 0.2 to 1.5.

117. The method of any one of items 115-116 wherein the carboxymethyl cellulose has a molecular weight in a range from 17,000 to 700,000.

118. The method of any one of items 113-117 wherein the active agent(s) is selected from the group consisting of analgesics, local anesthetics, anti-acne agents, anti-angina agents, antiarrhythmics, antibacterials, anti-convulsives, antidepressants, anti-rheumatics, sex hormones, anti-fungals, anti-hypertensives, anti-hypothyroid agents, anti-malarials, anti-migraine agents, anti-nausea agents, skin lighteners, dopamine receptor antagonists, muscle relaxants, sclerosing agents, vitamins and combinations thereof.

119. The method of any one of items 113-118 wherein the crystallization inhibitor is polyvinylpyrrolidone (PVP).

120. The method of item 119 wherein the concentration of the polyvinylpyrrolidone (PVP) is 0.5% to 20%.

121. The method of any one of items 113-120 wherein the vehicle includes a polyhydric alcohol.

122. The method of item 121 wherein the polyhydric alcohol is selected from the group consisting of propylene glycols, glycerols, polyethylene glycols and combinations thereof.

123. The method of any one of items 121-122 wherein the concentration of the polyhydric alcohol is from 0.5% to 20%.

124. The method of any one of items 113-123 wherein the adhesive composition exhibits a Peel on polyethylene value of at least 0.5 N/in.

125. The method of item 124 wherein the adhesive composition exhibits a Peel on polyethylene value of at least 1.5 N/in.

126. The method of item 125 wherein the adhesive composition exhibits a Peel on polyethylene value of at least 2.5 N/in.

127. The method of any one of items 113-126 wherein the adhesive composition exhibits a Tack value of at least 10 N/in.

128. The method of item 127 wherein the adhesive composition exhibits a Tack value of at least 25 N/in.

129. The method of item 128 wherein the adhesive composition exhibits a Tack value of at least 40 N/in.

130. The method of any one of items 113-129 wherein the incorporating includes bringing the adhesive composition to a temperature less than 75° C.

131. The method of item 130 wherein the temperature is between 60° C and 70° C.

**Claims**

1. An adhesive composition comprising:

   20% to 90% by weight of an adhesive;
   0.1% to 50% by weight of at least one absorbent;
   0.1% to 20% by weight of at least one active agent; and
   0% to 30% by weight of at least one crystallization inhibitor.

2. The adhesive composition of claim 1, further comprising 0.1% by weight to 30% by weight of a vehicle.

3. The adhesive composition of claim 1, wherein the at least one active agent exists in a stable amorphous state within the adhesive composition.

4. The adhesive composition of claim 1, wherein the various components are homogeneously blended into a single cohesive mass.

5. The adhesive composition of claim 1, wherein the at least one absorbent is selected from the group consisting of (i) insoluble swellable polymers, (ii) hydratable polymers, (iii) water soluble polymers, (iv) synthetic absorbents, (v) super absorbent polymers, and (vi) combinations of any one or more of (i) - (v).

6. The adhesive composition of claim 1, wherein the at least one absorbent is carboxymethyl cellulose.

7. The adhesive composition of claim 1, wherein the at least one crystallization inhibitor is soluble.

8. The adhesive composition of claim 1, wherein the at least one crystallization inhibitor is polyvinylpyrrolidone.

9. The adhesive composition of claim 1, wherein the at least one active agent is selected from the group consisting of analgesics, local anesthetics, anti-acne agents, anti-angina agents, antiarrhythmics, antibacterials, anti-convulsives, antidepressants, anti-rheumatics, sex hormones, anti-fungals, anti-hypertensives, anti-hypothyroid agents, anti-malarials, anti-migraine agents, anti-nausea agents, skin lighteners, dopamine receptor antagonists, muscle relaxants, sclerosing agents, vitamins, and combinations thereof.

10. The adhesive composition of claim 2, wherein the vehicle includes a polyhydric alcohol, preferably wherein the polyhydric alcohol is selected from the group consisting of propylene glycols, glycerols, polyethylene glycols, and combinations thereof.

11. The adhesive composition of claim 1, wherein the adhesive composition exhibits a Peel on polyethylene value of at least 0.197 N/cm (0.5 N/in), preferably at least 0.591 N/cm (1.5 N/in), and/or wherein the adhesive composition exhibits a static absorption of at least 50 $g/m^2$/24 hours.

12. An article adapted to be adhered to biological skin and release at least one active agent to the skin, the article defining a surface and comprising the adhesive composition of claim 1 disposed on at least a portion of the surface.

13. A method of releasing at least one active agent to a region of interest on biological skin, the method comprising:

    providing an article adapted for placement along biological skin, the article defining at least one face;
    providing the adhesive composition of claim 1;
    depositing the adhesive composition onto the face of the article; and
    applying the article to a region of interest on biological skin, whereby the adhesive composition is directed toward and overlies the region of interest,
    preferably wherein the adhesive composition contacts at least a portion of the region of interest on biological skin.

14. A method of increasing at least one of (i) extent of release and (ii) rate of release of at least one active agent from the adhesive composition of claim 1, the method comprising:
    incorporating the at least one absorbent in the adhesive composition, whereby the extent and/or rate of release of the at least one active agent is increased.

**15.** The method of claim 14, wherein the active agent is ibuprofen, and upon increasing the amount of absorbent in the adhesive composition by a factor of 2.0, the extent of release of ibuprofen is increased by a factor greater than 2.0.

FIG. 1

FIG. 2

FIG. 3

MAIN EFFECTS PLOT FOR ELUTION 2 HR
DATA MEANS

FIG. 4

MAIN EFFECTS PLOT FOR ELUTION 6 HR
DATA MEANS

FIG. 5

Europäisches
Patentamt

European
Patent Office

Office européen
des brevets

# EUROPEAN SEARCH REPORT

Application Number

EP 22 20 7478

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br>A | EP 0 764 441 A1 (OREAL [FR])<br>26 March 1997 (1997-03-26)<br>* page 1, lines 3-5, 53-57 *<br>* page 3, lines 9-14, 24-25 *<br>* page 4, lines 28-37, 43-47 *<br>* page 6; example 1 * | 2,4-7,<br>9-11,15<br>3,8 | INV.<br>A61K9/70<br>A61K47/10<br>A61K47/32<br>A61K31/192 |
| X<br>Y<br>A | EP 2 110 125 A1 (NITTO DENKO CORP [JP])<br>21 October 2009 (2009-10-21)<br>* page 2, paragraphs 1, 6-7 *<br>* page 3, paragraph 11-12 *<br>* page 4, paragraph 21 *<br>* page 6, paragraph 33 *<br>* Composition A;<br>page 8; table 1 *<br>* page 9; example 1 * | 1,12-14<br>2,4-7,<br>9-11,15<br>3,8 | |
| Y<br><br><br><br>A | WO 02/087645 A1 (AV TOPCHIEV INST<br>PETROCHEMICAL [RU]; CLEARY GARY W [US];<br>PARANDOOSH SH)<br>7 November 2002 (2002-11-07)<br>* page 3, lines 5-33 *<br>* page 9, lines 19-36 *<br>* page 10, lines 1-25 *<br>* page 13, lines 14-27 *<br>* page 21, lines 30-36 *<br>* pages 22-23 *<br>* page 24, lines 1-20 *<br>* page 32, lines 6-12, 31-35 * | 2,4-7,<br>9-11,15<br><br>3,8 | TECHNICAL FIELDS SEARCHED (IPC)<br><br>A61K |
| Y<br><br>A | US 6 280 765 B1 (GUERET JEAN-LOUIS H [FR])<br>28 August 2001 (2001-08-28)<br>* column 1, lines 9-14 *<br>* column 2, lines 55-62 *<br>* column 3, lines 39-50 *<br>* column 5, lines 5-31, 38-65 * | 2,4-7,<br>9-11,15<br>3,8 | |

The present search report has been drawn up for all claims

| Place of search | Date of completion of the search | Examiner |
|---|---|---|
| The Hague | 28 February 2023 | Raposo, Antonio |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                         

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 20 7478

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2023

| Patent document cited in search report | | Publication date | Patent family member(s) | | Publication date |
|---|---|---|---|---|---|
| EP 0764441 | A1 | 26-03-1997 | CA | 2186042 A1 | 21-03-1997 |
| | | | DE | 69612345 T2 | 25-04-2002 |
| | | | EP | 0764441 A1 | 26-03-1997 |
| | | | ES | 2156264 T3 | 16-06-2001 |
| | | | FR | 2738744 A1 | 21-03-1997 |
| | | | JP | 3051063 B2 | 12-06-2000 |
| | | | JP | H09124469 A | 13-05-1997 |
| EP 2110125 | A1 | 21-10-2009 | AT | 499930 T | 15-03-2011 |
| | | | CA | 2662499 A1 | 16-10-2009 |
| | | | CN | 101574332 A | 11-11-2009 |
| | | | EP | 2110125 A1 | 21-10-2009 |
| | | | JP | 5552255 B2 | 16-07-2014 |
| | | | JP | 2009275040 A | 26-11-2009 |
| | | | US | 2009264806 A1 | 22-10-2009 |
| WO 02087645 | A1 | 07-11-2002 | AT | 438418 T | 15-08-2009 |
| | | | AU | 2002308612 B2 | 10-04-2008 |
| | | | CA | 2445086 A1 | 07-11-2002 |
| | | | EP | 1390085 A1 | 25-02-2004 |
| | | | ES | 2331302 T3 | 29-12-2009 |
| | | | JP | 4116447 B2 | 09-07-2008 |
| | | | JP | 2004536898 A | 09-12-2004 |
| | | | RU | 2276998 C2 | 27-05-2006 |
| | | | US | 2003170308 A1 | 11-09-2003 |
| | | | US | 2009258060 A1 | 15-10-2009 |
| | | | US | 2013261526 A1 | 03-10-2013 |
| | | | US | 2014371692 A1 | 18-12-2014 |
| | | | WO | 02087645 A1 | 07-11-2002 |
| US 6280765 | B1 | 28-08-2001 | AR | 011710 A1 | 30-08-2000 |
| | | | CA | 2232616 A1 | 11-10-1998 |
| | | | DE | 69830095 T2 | 19-01-2006 |
| | | | EP | 0870498 A1 | 14-10-1998 |
| | | | ES | 2245017 T3 | 16-12-2005 |
| | | | FR | 2761889 A1 | 16-10-1998 |
| | | | JP | 2865659 B2 | 08-03-1999 |
| | | | JP | H10287559 A | 27-10-1998 |
| | | | US | 6280765 B1 | 28-08-2001 |

EPO FORM P0459

For more details about this annex : see Official Journal of the European Patent Office, No. 12/82

**REFERENCES CITED IN THE DESCRIPTION**

**Patent documents cited in the description**

- US 61778620